# EUROPEAN PATENT APPLICATION

(11) **EP 4 632 663 A1**
(43) Date of publication of application: **15.10.2025**
(21) Application number: 24382361.4
(22) Date of filing: 09.04.2024

(54) **ROBOTIZED LAPAROSCOPIC SURGICAL SYSTEM WITH AUGMENTED REALITY**

(71) Applicant: Rob Surgical Systems, SL, 08820 El Prat de Llobregat (ES)
(72) Inventor: AMAT GIRBAU, Josep, 08023 Barcelona (ES)
(74) Representative: Torner, Juncosa I Associats, SL

(57) **Abstract**

A robotized laparoscopic surgical system with augmented reality comprising a laparoscopic camera for acquiring, in real time, an image of a first type; and a control unit for receiving the image of the first type and superimposing it over an image of a second type. The control unit, prior to the superposition, carries out a preprocessing of the two types of images, comprising: obtaining a series of discrete points in the images; defining a number of reference triangles using the series of discrete points obtained; and calculating a spatial position and orientation of the reference triangles by applying, to a center of gravity of the triangle, in a direction, a vector that is proportional to the area of the triangle; and projecting the reference triangles of the image of the second type following a Z axis of the camera coordinates and projecting back the obtained image on a two-dimensional plane.

## Description

### Field of the Invention

This invention generally concerns the field of robotized laparoscopic surgery. In particular, the invention refers to a robotized laparoscopic surgical system with augmented reality.

### Background of the Invention

Current robotized laparoscopic surgery techniques offer the possibility of carrying out highly complex interventions with exceptional precision, having significant advantages in various surgical specialties. This approach is highly beneficial in cases where the access to the operational field is particularly difficult.

In this minimally invasive surgical modality, robotic arms are used to control instruments and specific tools, as well as to insert and guide a camera providing a detailed view of the intervention area. These robotic arms are remotely controlled by the surgeon or medical staff through a control console provided with a screen that allows monitoring the scene in real time.

In addition to the surgical precision inherent to the use of robotics, the information technology associated with the control of the robotic arms allows for the integration of various aids, enhancing the ability of the surgeon by providing additional information and, as a result, increasing the safety of the patient by ensuring greater quality when carrying out the intervention.

This invention is of particular application in this type of minimally invasive surgery, carried out by means of small incisions in the patient while the scene is being viewed on the screen, which not only serves as a means to display the surgical scenario, but it may also be used to offer additional information to the surgeon, without modifying the conventional operational procedure. This way, visual aids may be provided, such as outlines, trajectories, indicia, or contrast agents, and even the ability to view hidden elements, thus increasing the safety of the surgical procedure.

In recent years, significant efforts have been devoted in the investigation for providing aids to the surgeon that will make up for the loss of the sense of touch during manual interventions, virtual reality standing out among these.

Virtual reality has been successfully integrated in formation trainers, where the surgeons may carry out exercises in highly realistic synthetic environments or by means of virtual environments. In these cases, by applying virtual images in a known synthetic environment, technical challenges associated with the implementation of the virtual reality in the surgical practice in real conditions are avoided.

In the academic setting, some scientific articles exist which address the application of virtual reality in simulators [1-6].

In addition, patents and patent applications are known related to simulators endowed with virtual reality, such as US5882206A, US7850456B2, and WO2014052158A3. In these patents, the generation of virtual images is described which realistically emulate a surgical field, as well as the inclusion of these synthetic images in a physical scenario simulating the surgical environment. Although these computing systems control the simulator, they do not require the ability to carry out the concurrence between virtual and physical realities, as they operate on one single common reference system.

The integration of virtual reality in robotized laparoscopic surgery systems has also been the object of study in numerous published academic papers [7-9] and in conferences, especially in events such as CRAS (Conference on New Technologies for Computer and Robot Assisted Surgery) or ICRA (IEEE Robotics and Automation Society).

In the setting of practical surgery applications, patent application WO2017066373A1 is also known, describing a method aimed at the navigation and combining real images of the scene with virtual images. However, this document does not address the challenge of the correspondence between the different reference systems in the images, a crucial aspect for operating in real time and being able to apply the technique in real-life working conditions.

Therefore, there exists the need to develop new robotized laparoscopic surgery systems with augmented reality which will allow the virtual reality system of assistance to the surgeon/medical staff to be executed in a very short time, making it operative in surgical environment in real-life conditions.

### References

[1] Virtual reality training for surgical trainees in laparoscopic surgery. Kurinchi Selvan Gurusamy et al. 21 January 2009
[2] Virtual reality simulators and training in laparoscopic surgery. Eugenia Yiannakopoulou et al. January 2015.
[3] An Evidence-Based Virtual Reality Training Program for Novice Laparoscopic Surgeons. Rajesh Aggarwal et al. August 2006.
[4] Virtual reality training in laparoscopic surgery: A systematic review & meta-analysis. Medhat Alaker et al. May 2016.
[5] Virtual reality training leads to faster adaptation to the novel psychomotor restrictions encountered by laparoscopic surgeons. J.A. Jordan et al. 15 July 2014.
[6] Virtual Reality Training in Laparoscopic Surgery: A Preliminary Assessment of Minimally Invasive Surgical Trainer Virtual Reality (MIST VR). A. G. Gallagher et al. 1999.
[7] The status of augmented reality in laparoscopic surgery as of 2016. Sylvain Berhardt et al. April 2017.
[8] Augmented Reality in Surgery. Jeffrey H. Shuhaiber. 1 February 2004.
[9] Augmented Reality for Robotics: A Review. Zhanat Makhataeva and Huseyin Atakan Varol. 2 April 2020.

### Disclosure of the Invention

To this end, the present invention provides a robotized laparoscopic surgical system enabling the surgeon/medical staff to view complementary images superimposed on the anatomy in real time, while he/she operates with real images of the scene. Similarly, the system may perceive the proximity or contact of the forceps with the anatomic elements of the operational field, such that it will be an aid to the guidance of the trajectories and/or it protect from undesirable movements that may harm the patient.

The invention consists of two complementary components: algorithms designed to refer to the reference axes of the information available with the reference axes of the anatomic elements present in the scene and of the images obtained by the laparoscopic camera, and a user interface that provides access to this information, as well as its selection, selective activation, manual positioning, and/or modification of the functions carried out automatically.

The algorithm implemented to achieve the superposition of images and preexisting data of the patient on the real-time images captured by the laparoscopic camera involves searching for the best match between the two three-dimensional images with unknown reference axes. This process entails a multi-parametric search of seven variables: three for position, three for orientation and one for scale factor. Without simplifications, this would require a search process of n⁷ order, which implies a long calculation time. The methodology and the algorithm developed in this invention allow for the necessary simplifications so that the virtual environment system of assistance to the surgeon/medical staff may be executed in a very short time, making it operative.

Embodiments of the present invention provide a robotized laparoscopic surgical system with augmented reality that comprises a laparoscopic camera configured for acquiring, in real time, at least one image of a first type from a patient; and a control unit including a memory and one or more processing units, the control unit being configured to receive the image of the first type and superimpose it over at least one image of a second type obtained from the patient, obtaining a virtual reality as a result.

According to the invention, the control unit, prior to the superposition of the image of the first type over the one of the second type, is configured to carry out a preprocessing of each one of the two types of images, said preprocessing comprising a) obtaining a series of discrete points in the images; b) defining a number of reference triangles using the series of discrete points obtained; and c) calculating a spatial position and orientation of the reference triangles by applying, to a center of gravity of the triangle, in a direction, a vector that is proportional to the area of the triangle; and projecting the reference triangles of the image of the second type following a Z axis of the laparoscopic camera coordinates and projecting back the image obtained on a two-dimensional plane.

Likewise, the superposition comprises relating similar triangles on the two types of images, adjusting a scale factor of the similar triangles, and carrying out several shape rotations or translations on similar triangles.

According to the present invention, the one or more images of the first type are two-dimensional images and the one or more images of the second type are three-dimensional images.

The one or more images of the second type may be generated by computerized tomography, nuclear medicine, magnetic resonance, or ultrasound, among others.

In some embodiments, in step c), the preprocessing further comprises removing the reference triangles comprising at least an angle whose value is greater than or equal to a specific threshold value.

In some embodiments, obtaining the series of discrete points comprises obtaining a first set of inspection points of the images by means of the application of a mesh onto the images and the intersection of said mesh with horizontal and vertical coordinates; defining a sphere on each inspection point of the first set of inspection points and applying a scale factor to each defined sphere; and defining as a discrete point the inspection points whose scale factor satisfies a specific criterion.

The criterion may consist of the Va/Vb ratio being greater or smaller than a given value, wherein Va corresponds to an inner volume and Vb to an outer volume of the voxels of each sphere

In some embodiments, the superposition is also carried out by modulating an intensity, color, and/or shade of the image of the first type.

In some embodiments, the control unit includes a number of control elements and a screen to display the virtual reality obtained.

Obtaining the series of discrete points, the definition of the reference triangles, the adjustment of the scale factor, and/or carrying out the rotations or translations may be carried out by means of the control elements.

In some embodiments, the system also includes electrosurgical forceps.

The control elements may be configured to detect signals corresponding to stresses exerted by the electrosurgical forceps, such that the virtual reality obtained is complemented with said stresses which are in turn transmitted to a user, e.g., a surgeon/medical staff, by means of the control elements.

In yet other embodiments, the control elements may be configured to transmit to the user signals corresponding to a haptic perception, such signals being generated from the virtual reality obtained or from restrictions, particularly mechanical, of the system.

In some embodiments, the system also includes a 3D display device operatively connected to the control unit and configured for displaying the virtual reality obtained.

Consequently, the present invention offers a system (and an associated method) for assisting a surgeon/medical staff during robotized laparoscopic surgical interventions by means of remote operation. This is achieved by presenting a virtual reality complementing the images provided to the surgeon/medical staff by the laparoscopic camera, with additional visual information derived from the examination of the patient. The additional visual information may be obtained applying a contrast agent to the patient or by the manual or automatic input of graphic instructions by specialized personnel, as well as collecting it from external databases.

One advantage the present invention provides is that it does not add any new physical elements to the robotic system or any additional sensors on the instrument, and allows assisting and guiding the surgeon/medical staff so that he/she may carry out the surgical operation with greater safety and efficiency.

### Brief Description of the Drawings

The foregoing and other characteristics and advantages will be more fully understood from the following detailed description of several exemplary embodiments, of merely illustrative and non-limiting nature, with reference to the attached drawings, wherein:
Fig. 1 illustrates a robotized laparoscopic surgical system, according to an embodiment of the present invention.
Fig. 2 schematically illustrates the steps for the processing of the information, according to an embodiment of the present invention.
Figs. 3A-3D schematically illustrate how the discrete points are calculated, according to an embodiment of the present invention.
Fig. 4 schematically illustrates the removal of some of the reference triangles, according to an embodiment of the present invention.
Fig. 5 illustrates the registering process followed to automatically carry out the superposition of the two types of images, according to an embodiment of the present invention.
Figs. 6 and 7 graphically illustrate, respectively, how the scale factor, and the position and orientation of the images to be superimposed may be modified.

### Detailed Description of the Invention and of exemplary embodiments

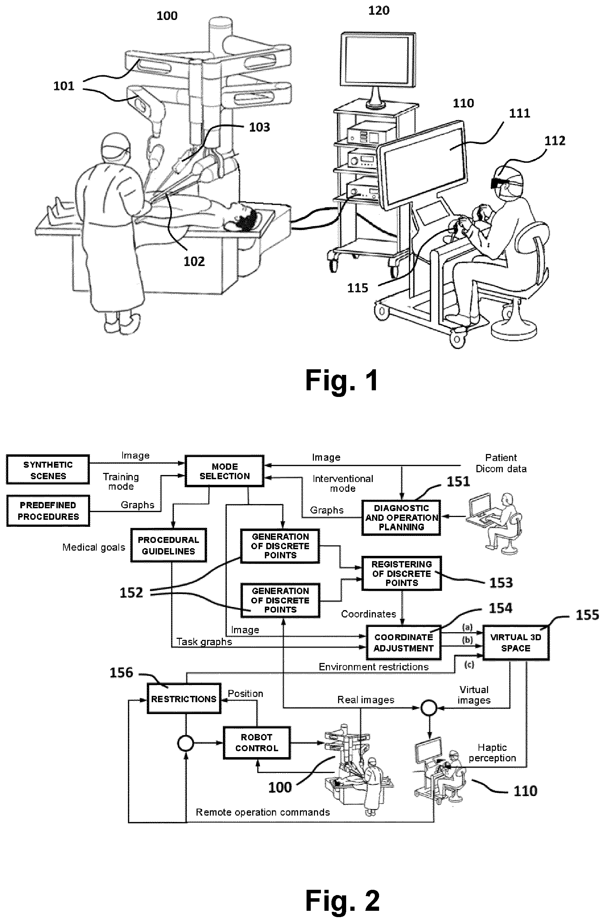

Fig. 1 illustrates a robotized laparoscopic surgical system, according to an embodiment of the present invention. This system is comprised of an actuation robotic system 100, a control unit 110 and a tower equipped with auxiliary devices 120, as respiratory assistance, lighting for laparoscopy or power supply for electro-cauterization procedures.

The robotic system 100 is provided with a number of robotic arms 101 allowing for the manipulation of a number of electrosurgical forceps 102 as well as a laparoscopic camera 103. The control unit 110 includes a display screen 111, and/or 3D display equipment 112, such as glasses, for viewing the generated virtual reality. In addition, the control unit 110 includes control elements 115 that allow a user (e.g. a surgeon) to handle the previous instruments/elements, as well as perceive the stresses applied by the electrosurgical forceps 102 on the body of the patient.

Fig. 2 illustrates an example of the processing of the information carried out by the one or more processing units of the control unit 110. In this example, in particular, the virtual environment is formed superimposing three components in one single three-dimensional space 155:
a) Images, acquired during the diagnostic and operation planning step, derived from the segmentation of an anatomic region of the patient, which will be subject to the intervention due to its pathology.
b) Images indicating trajectories or limits of the task to be carried out 151, input by a specialist 180 in the operation diagnostic and planning phase.
c) Images or signs related to movement restrictions or limits in the task 156, due to limiting conditions of the robotic arms 101, the instruments or possible collisions.

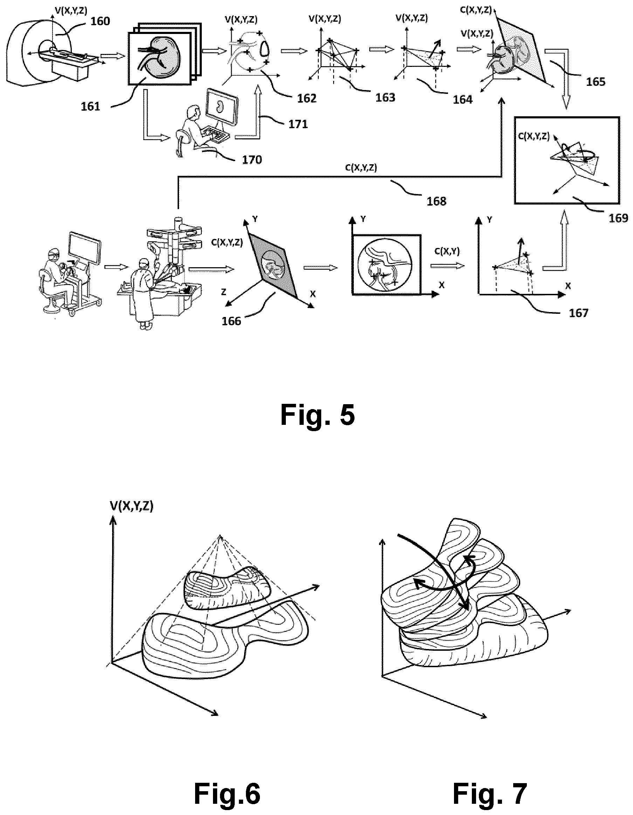

Images a) and b) make reference to the coordinate axes of the equipment used in the step of examination and diagnostic of the patient 160 (see Fig. 5) and must make reference to the coordinate axes 154 of the images of a first, real type, obtained from the anatomy by means of the laparoscopic camera 103. In order to achieve this, a calculation algorithm is applied which determines the three translations, the three rotations and the scale adjustment to achieve the best fit between images referred to different reference axes.

The automatic registering may be used by the surgeon to carry out a first match of the images he/she wishes to view over the images of the first type obtained by the laparoscopic camera 103. This automatic process may operate in three phases. In a first phase a small set of discrete points from the images 152 is obtained which will define a number of reference triangles, to thus reduce the calculation time; in a second phase an adjustment of the scale factor between images is carried out; and in the third phase the position and orientation registering is effected, solely based on a number of discrete points 153.

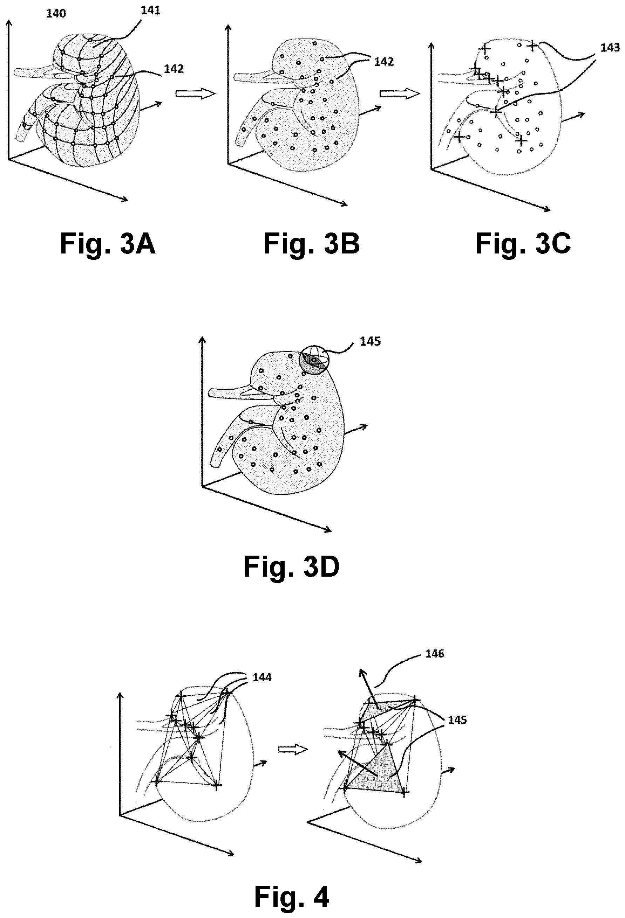

With reference now to Figs. 3A-3D, the present invention uses computer-aided viewing techniques in order to obtain discrete points 143. Although obtaining discrete points is a known technique, the conventional methods generate dense point clouds, which results in long calculation times for the registering. In this context, the present invention carries out a preselection of a set of points (see Fig. 3A) by means of the application of an initial mesh 141 on the image. This mesh ensures a uniform distribution of preselected inspection points 142 on the displayed surface (Fig. 3B). Then, discrete points 143 are obtained by means of the removal of the points that are not considered discrete (Fig. 3C).

In particular, in this invention, those inspection points 142 where, within a sphere 145 centered on each inspection point (Fig. 3D), the two voxel regions formed -the inner Va and outer Vb volumes- have a Va/Vb ratio greater or smaller than a predetermined value are classified as discrete points 143. For example, in two-dimensional images, the spherical environments of each inspection point will be circular environments.

In some embodiments, with the aim of achieving very short registering times, the calculation of the best match between images is carried out considering the spatial position and orientation of the triangles formed by these discrete points (Fig. 4). In order to reduce the number of triangles that may be formed with the connection of each discrete point 143 with all others 144, those in which one of their angles is greater than or equal to a specific threshold value are not considered useful triangles. This way, the different parts of an object of the scene will be defined by the triangles considered useful 145 that define the size and spatial orientation of each element.

An example of the registering process followed to automatically carry out the superposition of the two types of images is presented in Fig. 5: two-dimensional images 166 obtained by the laparoscopic camera 103 and the three-dimensional images 161 acquired by means of any medical image specialty, such as computerized tomography, nuclear medicine, magnetic resonance, ultrasound, fluorescence, etc.

According to the invention, the superposition may further comprise the modulation of the intensity, color, and/or shade of the image of the first type.

In the images of the second type 161, discrete points 162 are obtained, and reference triangles 163 are defined using these discrete points. It is important to highlight that, in some embodiments, only the resulting triangles 164 are used after removing those whose angle is greater than or equal to said threshold value, more in particular, the obtuse triangles. This same process is applied to collect the discrete points from the images of the first type, generating the reference triangles 167 as a result.

In order to carry out the registering between the three-dimensional space triangles and those of the two-dimensional space, a projection of the coordinates C(XYZ) of the laparoscopic camera 103 is obtained of the three-dimensional space triangles 164 over the two-dimensional plane 165 following the Z axis. This provides the registering between triangles using the same coordinate axes 170 and operates in three phases. In the first phase, the triangles which must be considered homologous due to the similarity between their three angles are related to one another. In the second phase the scale factor is adjusted to obtain the smallest surface difference between homologous triangles. Finally, in the third phase, shape rotations are carried out minimizing the different orientations between homologous triangles, their orientation being considered to be the vector perpendicular to their longest edge.

This way, the registering process may be carried out between either three-dimensional or two-dimensional images of equal dimensions, and may also be carried out between three-dimensional V(XYZ) or two-dimensional C(XY) images. Therefore, the processing effected is carried out in a very short time, since no n⁷ operations are needed.

In some cases, due to the complexity of the scene, the automatic registering process may not always provide a perfect superposition. For this reason, in some embodiments, the ability to manually carry out adjustments or a repositioning with the control elements 115 themselves of the remote operation station 110 is included. In order to facilitate this manual operation, this adjustment of scale factor and of orientation may be carried out simultaneously. To activate the manual adjustment mode, the surgeon is provided with a button, a pedal, and/or a selection and control menu in the control console. The surgeon may modify, with one of the controls, the scale factor of the images to be superimposed (see Fig. 6) and, with the other, position and orient the image in all three dimensions (see Fig. 7). This is done just as easily as the surgical instruments are controlled.

Likewise, during the manual adjustment, the surgeon may be provided with a menu on the screen which allows for the input of points, fixing them, modifying their position and erasing them, when convenient, and once validated, carrying out an automatic registering with these manually fixed points and later correcting them at his/her discretion.

The virtual reality created in the control unit 110 may be complemented with the stresses perceived with the control elements 115. This allows not only perceiving the movement limits, but also haptically sensing the correct routing of the programmed trajectories during the previous planning step. In addition, in some examples, these stresses may be combined with other geometric restrictions, such as those corresponding to the movement limits of the robot that the operator controls remotely or any other geometric restrictions 156 introduced for safety reasons.

In yet another embodiment, the present invention provides a method for generating an augmented reality by means of a robotized laparoscopic surgical system. The method comprises:
acquiring, from a patient, at least one image of a first type using the laparoscopic camera;
acquiring or receiving at least one image of a second type;
carrying out a preprocessing of each one of the two types of images, comprising: obtaining a series of discrete points in the images; defining a number of reference triangles using the series of discrete points obtained; and calculating a spatial position and orientation of the reference triangles by applying, to a center of gravity of the triangle, in a direction, a vector that is proportional to the area of the triangle; projecting the reference triangles of the image of the second type following a Z axis of the laparoscopic camera coordinates and projecting back the obtained image on a two-dimensional plane;
obtaining a virtual reality by means of the superposition of the image of the first type over the image of the second type, wherein the superposition comprises: relating similar triangles in the two types of images, adjusting a scale factor of the similar triangles, and carrying out several shape rotations or translations on the similar triangles.

Despite it having been previously illustrated and described with reference to certain specific embodiments, the present invention does not intend to be limited to the details shown. On the contrary, various modifications in the details may be introduced within the scope and the equivalence range of the claims.

The scope of the present invention is defined in the attached claims.

## Claims

1. A robotized laparoscopic surgical system with augmented reality, comprising:
a laparoscopic camera (103) configured to acquire, in real time, at least one image of a first type (166) from a patient; and
a control unit (110) including a memory and one or more processing units, the control unit (110) being configured to receive the image of the first type (166) and to superimpose it over at least one image of a second type (161) obtained from the patient, obtaining a virtual reality (169) as a result;
**characterized in that** the control unit (110), prior to the superposition of the image of the first type (166) over that of the second type (16), is configured to:
- carry out a preprocessing of each one of the two types of images (161, 166), said preprocessing comprising:
a) obtaining a series of discrete points (143, 162) in the images;
b) defining a number of reference triangles (163) using the series of discrete points (143, 162) obtained; and
c) calculating a spatial position and orientation of the reference triangles by applying, to a center of gravity of the triangle, in a direction, a vector that is proportional to the area of the triangle; and
- project the reference triangles of the image of the second type following a Z axis of the coordinates C(XYZ) of the laparoscopic camera (103) and project back the image obtained on a two-dimensional plane;
**and in that** the superposition further comprises:
relating similar triangles in the two types of images,
adjusting a scale factor of the similar triangles, and
carrying out several shape rotations or translations on similar triangles.

2. The system of claim 1, wherein in step c), the preprocessing further comprises removing the reference triangles comprising at least one angle whose value is greater than or equal to a given threshold value.

3. The system of claim 1 or 2, wherein obtaining the series of discrete points (143) comprises:
obtaining a first set of inspection points (142) of the images by means of the application of a mesh (141) onto the images and the intersection of said mesh (141) with horizontal and vertical coordinates;
defining a sphere (145) on each inspection point (142) of the first set of inspection points and applying a scale factor to each defined sphere (145);
defining as a discrete point (143) the inspection points (142) whose scale factor satisfies a given criterion.

4. The system of claim 3, wherein the criterion consists of the Va/Vb ratio being greater or smaller than a given value, where Va corresponds to an inner volume and Vb to an outer volume of the voxels of each sphere (145).

5. The system of any one of the previous claims, wherein the superposition is further carried out by modulating an intensity, color, and/or shade of the image of the first type.

6. The system of any one of the previous claims, wherein the control unit (110) includes a number of control elements (115) and a screen (111) configured to display the virtual reality obtained.

7. The system of claim 6, wherein the obtaining of the series of discrete points (143, 162), the definition of the reference triangles (163), the adjustment of the scale factor, and/or the carrying out of the rotations or translations is performed by means of the control elements (115).

8. The system of claim 6 or 7, further comprising electrosurgical forceps, wherein the control elements (115) are configured to detect signals corresponding to stresses applied by the electrosurgical forceps (102), such that the virtual reality obtained is complemented with said stresses that are in turn transmitted to a user using the control elements (115).

9. The system of claim 6 or 7, wherein the control elements (115) are configured to transmit to a user signals corresponding to a haptic perception, wherein the signals are generated from the obtained virtual reality or from restrictions (156) of the system.

10. The system of any one of the previous claims, further comprising a 3D display device (112), operatively connected to the control unit (110) and configured to display the virtual reality obtained.

11. The system of any one of the previous claims, wherein the image of the first type is a two-dimensional image and the image of the second type is a three-dimensional image.

12. The system of any one of the previous claims, wherein the image of the second type comprises a computerized tomography, nuclear medicine, magnetic resonance, or ultrasound image.
